# EUROPÄISCHE PATENTSCHRIFT

(11) **EP 0 568 797 B1**
(45) Veröffentlichungstag und Bekanntmachung des Hinweises auf die Patenterteilung: **15.12.1999**
(21) Anmeldenummer: 93104120.6
(22) Anmeldetag: 13.03.1993
(51) Int. Cl.: G01N 33/546, G01N 33/86

(54) **Verfahren zur Analyse partikelverstärkter Agglutinationsreaktionen auf Zentrifugalanalysatoren durch Bestimmung der Trübungsaufhellung**
Method for the analysis of particle agglutination by measurement of turbidity clearance using a centrifugal analyzer
Procédé pour l'analyse de l'agglutination de particules par mesure de l'élimination de la turbidité effectué à l'aide d'un analyseur à centrifugation

(30) Priorität: 04.04.1992 DE 4211351
(43) Veröffentlichungstag der Anmeldung: 10.11.1993
(73) Patentinhaber: Dade Behring Marburg GmbH, 35001 Marburg (DE)
(72) Erfinder: Kraus, Michael, W-3550 Marburg (DE)

(56) Entgegenhaltungen:
- EP-A- 0 080 614
- EP-A- 0 317 796
- US-A- 4 208 185
- US-A- 4 720 465
- US-A- 4 988 630
- JOURNAL OF IMMUNOLOGICAL METHODS Bd. 89, 1986, NEW YORK US Seiten 257 - 263 B.B.LENTRICHIA ET AL. 'A sensitive kinetic latex agglutination immunoassay adapted to centrifugal analysis'
- PATENT ABSTRACTS OF JAPAN vol. 012, no. 307 (P-747)22. August 1988 & JP-A-63 078066 ( YATORON KK ) 8. April 1988

## Beschreibung

Die Erfindung betrifft ein Verfahren zur Bestimmung von Analyten, wobei eine Probe eines biologischen Materials, die möglicherweise diesen Analyten enthält, mit mindestens einem für den Analyten spezifischen Bindungspartner, der an einem partikelförmigen Trägermaterial immobilisiert ist, inkubiert wird und die durch den Analyten bewirkte Veränderung der Trübung bestimmt wird.

Immunchemische Nachweisverfahren zur quantitativen und qualitativen Bestimmung von therapeutisch und/oder diagnostisch wichtigen Substanzen basieren auf dem Festphasen-Prinzip, wobei auf einem wasserunlöslichen Träger (Festphase) ein zu dem nachzuweisenden Analyten affiner Bindungspartner (z.B. Antikörper, Antigen, Substrat und -analoga, Enzyme, Lectine) immobilisiert wird. Als Festphasen werden verschiedene, wasserunlösliche Polymere verwendet, die in verschiedenen geometrischen Ausführungsformen, wie Röhren, Kugeln oder Mikrotitrationsplatten zur Anwendung kommen.

Sogenannte heterogene Immunoassays, z.B. ELISA, erlauben durch die Bildung von Komplexen aus dem Analyt mit dem immobilisierten Bindungspartner, die Abtrennung von nicht gebundenen Analyten. In einem nachfolgenden Detektionsschritt wird der immobilisierte Analyt quantitativ bestimmt. Diese Verfahren sind jedoch arbeits- und zeitaufwendig.

In den sogenannten homogenen Verfahren werden vorwiegend nephelometrische oder turbidimetrische Methoden angewendet. Die Kinetik der Bildung von AB aus Analyt (A) und Bindungspartner (B) kann durch die dabei auftretende Veränderung der Streuung, bzw. Absorption des eingestrahlten Lichts verfolgt werden. Bei der Verwendung von suspendierbaren Partikeln, wie z. B. Latexpartikel, an die ein Bindungspartner immobilisiert ist, tritt eine Verstärkung der Effekte ein, wodurch die Bestimmung wesentlich niedrigerer Analytkonzentrationen ermöglicht wird. Diese homogenen Methoden sind schnell und einfach durchführbar und erlauben insbesondere die Automatisierung von Probenanalysen.

Die Bezeichung AB soll nicht eine Mengenzusammensetzung in einem bestimmten stöchiometrischen Verhältnis zueinander andeuten, sondern steht vielmehr für das bei der Reaktion von Analyt (A) und Bindungspartner, bzw. immobilisiertem Bindungspartner (B) entstehende Produkt (AB).

Solche partikelverstärkte, turbidimetrische Immunoassays werden z. B. in der Patentschrift DE 29 05 434 beschrieben. Es werden darin Partikel mit einem Durchmesser < 1,6 µm verwendet, die in Konzentrationen von-0,05 - 1 % in der zu untersuchenden Lösung eingesetzt werden. Idealerweise wird nach diesem Patent die Reaktionskinetik bei Wellenlängen verfolgt, die mindestens das 1,5-fache der Partikeldurchmesser betragen. Die in einer Lösung gemessene Trübung zeigt in Abhängigkeit von der Partikelgröße ein Optimum: mit zunehmender Partikelgröße nimmt die Trübung zunächst zu, bei sehr großen Partikeln hingegen wieder ab (siehe auch: Malinski, J.A. und Nelsestuen, G.L., "Relationship of turbidity to the stages of platelet aggregation." Biochim. Biophys. Acta 882: 177-182 (1986)). Die Trübungsabnahme ist darauf zurückzuführen, daß bei Annäherung der Partikelgröße an die Wellenlänge des eingestrahlten Lichts die Streuung in Richtung des Lichtweges zunimmt und daher in turbidimetrischen Verfahren eine optische Aufhellung simuliert wird. Dieser Effekt wird bei nephelometrischen Verfahren durch Ausblenden des vorwärts gerichteten Streulichts unterdrückt.

In turbidimetrischen Verfahren wird bisher fast ausschließlich die Absorptionszunahme, also der ansteigende Teil der unterliegenden Funktion, zur Bestimmung von Analytkonzentrationen genutzt (siehe z.B.: Brustolin D. et al. "Immunoturbidimetric method for routine determinations of apolipoproteins A-1 and B.", Clin. Chem. 37(5), 742-747 (1991)). Zur Vermeidung des Effekts der Lichtstreuung in Vorwärtsrichtung wird daher bevorzugt langwelliges Licht, wie z.B. in der zitierten Patentschrift DE 2 905 434 >600 nm, verwendet.

Die Bestimmung von AB durch Verwendung der Aufhellungsreaktion (absteigender Teil der Funktion) wurde nur vereinzelt beschrieben (z. B. bei Dezelic, G.J. et al., "Latex Particle Agglutination in the Immunochemical System Human Serum Albumin/Anti-Human Serum Albumin Rabbit Serum", Eur. J. Biochem. 20(4), 553-560 (1971)). Die beschriebene Methodik erfordert zur Bestimmung der Geschwindigkeit der Trübungsabnahme jedoch sehr geringe Konzentrationen des sensibilisierten Latex im Bereich von 0,007 bis 0,028 %. Aufgrund dieser geringen Konzentrationen ist es notwendig, trübungsverursachende Verunreinigungen zu entfernen. Dies und die geringe Latexkonzentration bedingt, daß die Antigen-Antikörper Reaktion unvermeidbar vermindert wird und daher Meßzeiten von 18 bis 20 h notwendig sind. Die resultierende Präzision, Reproduzierbarkeit und Sensitivität dieser Methoden ist für die Bestimmung von Antigen- oder Antikörperkonzentrationen ungenügend (Cohen, R.J. and Benedek G.B., "Immunoassay by Light Scattering Spectroscopy", Immunochem. 12, 349-351 (1975)).

Die Entwicklung von Zentrifugalanalysatoren in der klinischen Chemie führte zur Adaptation turbidimetrischer Assays auf diesen Geräten, die eine automatische Abarbeitung und damit auch bessere Präzision der Messung erlaubten. Auch auf diesen Geräten wurden bisher nur Verfahren angewandt, in der der Analyt durch die bei der AB-Bildung bedingte Absorptionszunahme, turbidimetrisch bestimmt wird (siehe z.B. Gail A. Hudson et al. "Twelve-protein immunoassay profile on the Cobas Fara", J. Clin. Lab. Anal. 1, 191-197 (1987)).

Lentrichia und Yeung ("A sensitive kinetic latex agglutination immunoassay adapted to centrifugal analysis", J. Immunol. Meth. 89, 257-263 (1986) publizierten ein Verfahren, zur kompetitiven Bestimmung von HCG an einem Zentrifugalanalysator, wobei die Autoren ca. 6 min nach der üblichen Zunahme der Absorption eine Abnahme des Meßsignals beobachteten. Dieser absteigende Teil der Reaktionskinetik wurde benutzt um mit handelsüblichen Latexreagenzien (Partikeldurchmesser 220 nm) einen HCG-Assay zu adaptieren. Die Bestimmung wurde bei einer Wellenlänge von 690 nm durchgeführt, die Latexkonzentration im Testansatz betrug 0,09 %. Die Meßdauer dieses Verfahrens beträgt jedoch ca. 30 min und ist damit den gängigen turbidimetrischen Verfahren unterlegen, die in der Regel nur ca. 3 bis 5 min benötigen. Zudem bedingt die gemischte Reaktionskinetik (zunächst Zunahme des Signals, dann Abnahme) die Gefahr von Fehlmessungen: unspezifische Trübungen etwa führen zu einer Verlängerung des ansteigenden Anteils der Reaktionskinetik und somit u. U. sogar zu einem negativen Signal. Daher konnte erst 8 min nach Beginn der Reaktion mit der Messung begonnen werden. Dieses Verfahren, das im weiteren als Stand der Technik bezeichnet wird, ist somit noch unbefriedigend.

US-A-4,720,465 beschreibt ein öhnliches Verfahren unter Anwendung einer Partikeldichte von 2,8% und einer Zentrifugalbeschleinigung von zwischen 33 und 66 x g.

Die der Erfindung zugrundeliegende Aufgabe bestand nun darin, eine photometrische Methode zu finden, die auf an sich bekannten automatischen Zentrifugalanalysatoren mit deren Hilfe die Konzentration eines Analyten durch die Bildungskinetik von AB mit großer Präzision und Reproduzierbarkeit quantitativ bestimmt werden kann und zwar innerhalb der auf solchen Automaten üblichen Zeit von etwa 3-8 min (Zugabe der Probe bis Ergebnisausgabe).

Überraschenderweise wurde gefunden, daß unter geeigneten Reaktionsbedingungen bereits unmittelbar nach Ablauf der durch die Probenzugabe bedingten Misch- und Verteilungsphase die Trübungsabnahme gemessen werden kann und ein eindeutiges Ergebnis nach etwa 2- 7 min erhalten werden kann.

Ohne daß wir den Gegenstand der Erfindung durch eine Erklärung des Mechanismus der Reaktion einschränken wollen, scheinen uns die folgenden Punkte im Sinne der Erfindung positiven Einfluß auf die Bildungskinetik von AB:
1. eine Erhöhung der Zentrifugalbeschleunigung oder die Verwendung von Partikeln höherer Dichte, z.B. aus Magnetit,
2. die Verwendung möglichst kugelförmiger Partikel, um den Stokeschen Radius zu reduzieren, sowie durch Zusätze im Medium, die, wie z.B. Glycerin, Polyethylenglykol, zu einer höheren Dichte führen,
3. eine Erhöhung der Partikeldichte im Ansatz und der Beladungsdichte der sensibilisierten Partikel.

Überraschenderweise zeigte sich, daß der Einsatz von Zentrifugalbeschleinigungen, die größer sind, als die im Stand der Technik verwendeten, dazu führt, daß die dabei auftretende Aufhellung der analysierten Lösung im Gegensatz zu dem im Stand der Technik beschriebenen Verhalten sehr schnell einsetzt. Die Geschwindigkeit und das Ausmaß der Absorptionsabnahme ist abhängig von der Konzentration des Analyten in der untersuchten Lösung.

Gegenstand der Erfindung ist somit ein Verfahren zur Bestimmung eines Analyten, wobei eine Probe eines biologischen Materials, das möglicherweise diesen Analyten enthält, mit mindestens einem für den Analyten spezifischen Bindungspartner, der an einem partikelförmigen Trägermaterial immobilisiert ist, inkubiert wird, und
a) die Reaktion unter Einfluß einer im Meßverlauf konstanten Zentrifugalbeschleunigung erfolgt, die zwischen 800 und 10.000 x g liegt,
b) die Partikeldichte im Meßansatz mindestens 0,1 Gew.% beträgt,
c) als Trägermaterial möglichst kugelförmige, partikuläre natürliche und/oder synthetische Polymere verwendet werden
d) die Reaktion in einem gepufferter Medium mit definierter Dichte und Viskosität erfolgt
e) die auftretende Trübungsaufhellung bereits maximal 1 Minute nach der Probenzugabe gemessen wird und
d) die Konzentration des Analyten durch Vergleich der für die Probe gemessenen Trübungsaufhellung mit unter identischen Bedingungen gemessenen Werten für Proben mit bekanntem Analytgehalt eindeutig bestimmt wird.

Bevorzugt ist dabei ein solches Verfahren, wobei Analyt und spezifischer Bindungspartner Partner einer immunchemischen Reaktion sind, besonders bevorzugt ist dabei ein Verfahren, wobei ein spezifischer Bindungspartner ein Antikörper gegen ein Protein oder ein Peptid aus dem Gerinnungs-, Fibrinolyse- oder Komplementsystem ist, ganz besonders bevorzugt ist dabei als spezifischer Bindungspartner ein Antikörper gegen das Fibrinspaltprodukt D-Dimer. Antikörper im Sinne dieser Erfindung sind poly- und monoklonale Antikörper und auch Antikörperfragmente und modifizierte Antikörper, wie z. B. bispezifische Antikörper.

Bevorzugt ist ferner ein solches Verfahren, in dem als partikelförmiges Trägermaterial Copolymerisate aus Styrol-Methacrylsäure oder Methacrylat-Methacrylsäure verwendet werden.

Bevorzugt ist außerdem ein solches Verfahren, wobei die Veränderung der Absorption in einem Winkel von 0° bis 180° zur Sedimentationsrichtung verfolgt werden kann.

Bevorzugt ist darüberhinaus ein solches Verfahren, wobei zur Messung der Absorptionsänderung Wellenlängen aus dem Bereich von 280 bis 900 nm verwendet werden.

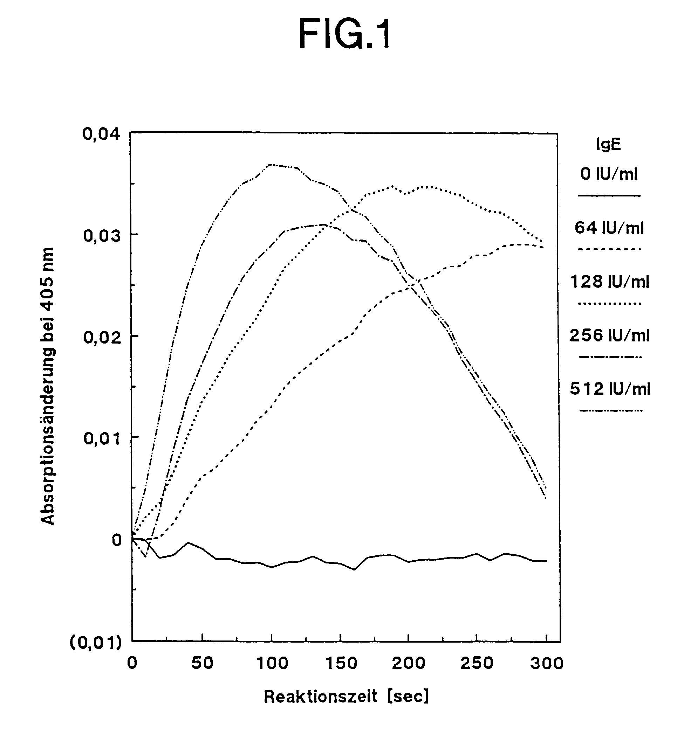

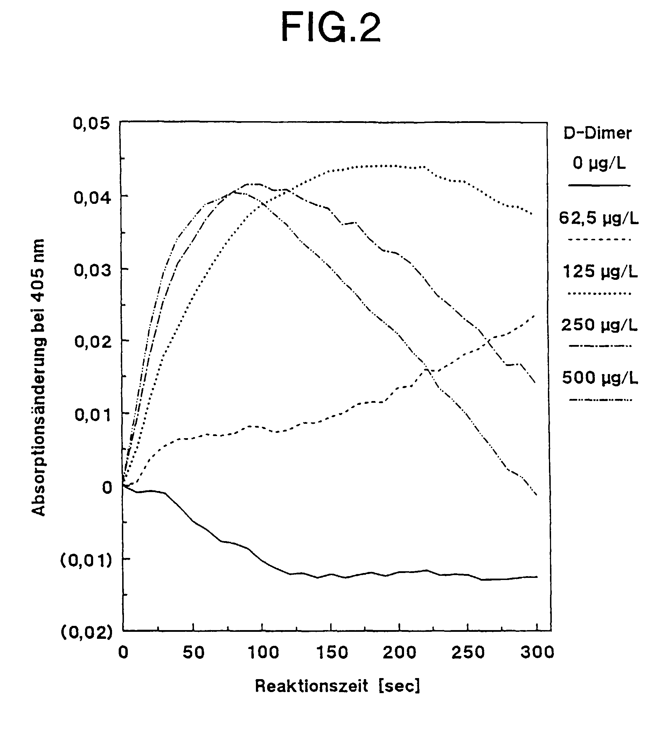

Bei Verwendung von Partikelkonzentrationen gemäß dem Stand der Technik, wie beispielsweise in Beispiel 2 (Figuren 1 und 2) ausgeführt mit 0,0125 Gew. % in der Reaktionslösung, zeigt sich der beschriebene Verlauf der Agglutinationsreaktion: Abhängig von der Analytkonzentration nimmt zunächst die Trübung der Lösung proportional zu. Diese Reaktionskinetiken wurden mit einem handelsüblichen Reagenz zur nephelometrischen Bestimmung von IgE (Figur 1), bzw. mit einem selbst hergestellten Latexreagenz zur Bestimmung von D-Dimer (wie in Beispiel 1 ausgeführt) erstellt. Die Partikeldurchmesser betrugen jeweils 230 nm.

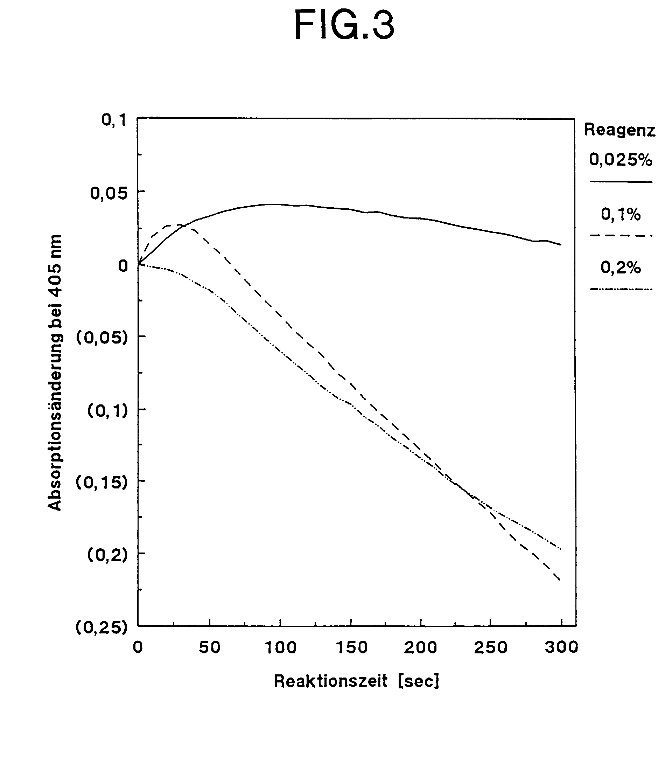

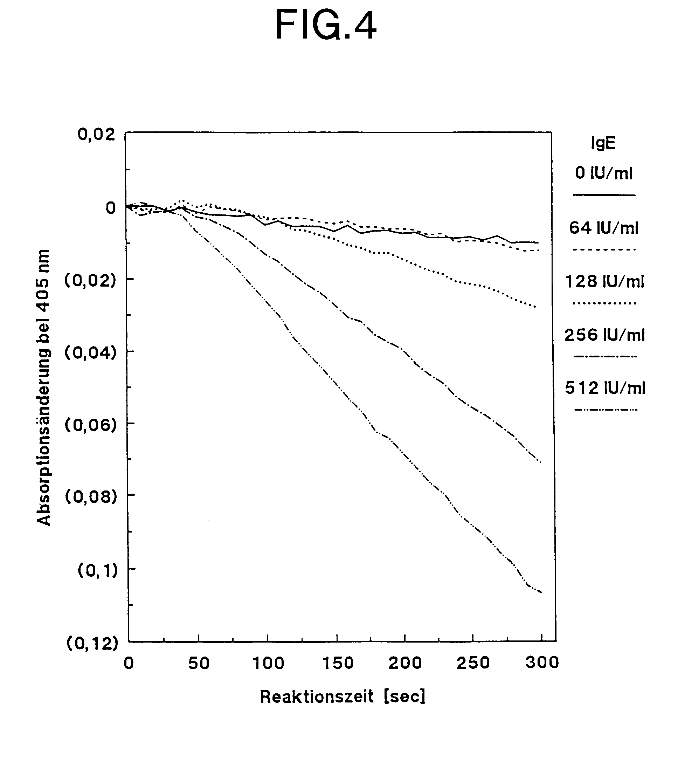

Verwendet man jedoch gemäß der vorliegenden Erfindung Partikeldichten, die oberhalb der im Stand der Technik verwendeten Konzentration von 0,09 % liegen, so ist weitgehend nur noch eine Trübungsabnahme zu verzeichnen. Bevorzugt wird dabei ein Bereich von 0,1 bis 1, besonders bevorzugt von 0,1 bis 0,4, ganz besonders bevorzugt von 0,1 bis 0,2 Gew %. Der Vergleich mit einer Latexkonzentration gemäß dem Stand der Technik ist beispielhaft in Figur 3 (Beispiel 3) für das D-Dimer Latexreagenz aufgezeigt. In den Figuren 4 (für das IgE-Latexreagenz) und 5 (für das D-Dimer Latexreagenz) ist dargestellt, daß bei höherer Partikelkonzentration gemäß der vorliegenden Erfindung, beispielsweise 0,1 % in der Reaktionslösung (Beispiel 4), die Reaktionskinetik bereits nach ca. 1 min bereits so konstant verläuft, daß Messungen innerhalb von 5 min möglich sind.

Bedingt durch die Mischvorgänge in der Reaktionsküvette von Zentrifugalanalysatoren kann üblicherweise nur mit einer gewissen zeitlichen Verzögerung nach Probenzugabe gemessen werden.

Zur Bestimmung der Analytkonzentrationen kann die kinetische Methode gewählt werden, d.h. die Rate der Absorptionsabnahme, die Endpunktmethode, d.h. die Bestimmung der absoluten Absorptionsdifferenz nach einem gewählten Zeitpunkt, oder die Schwellwertmethode, d.h. die Bestimmung der Zeit, bis eine bestimmte Absorptionsänderung eingetreten ist. Diese Möglichkeiten sind in Beispiel 6 in den Tabellen 1 und 2 exemplarisch dargestellt.

Überraschenderweise zeigte sich beim Vergleich von turbidimetrischen Verfahren mit Trübungszunahme und dem erfindungsgemäßen Verfahren (Vergleich der Figuren 1 und 4, sowie der Figuren 2 und 5), daß die absoluten Änderungen der Absorption in dem erfindungsgemäßen Verfahren um den Faktor 3 bis 5 höher liegen, was in der Regel mit einer entsprechenden Verbesserung der Meßgenauigkeit verbunden ist, was einen großen Vorteil der erfindungsgemäßen Methode darstellt.

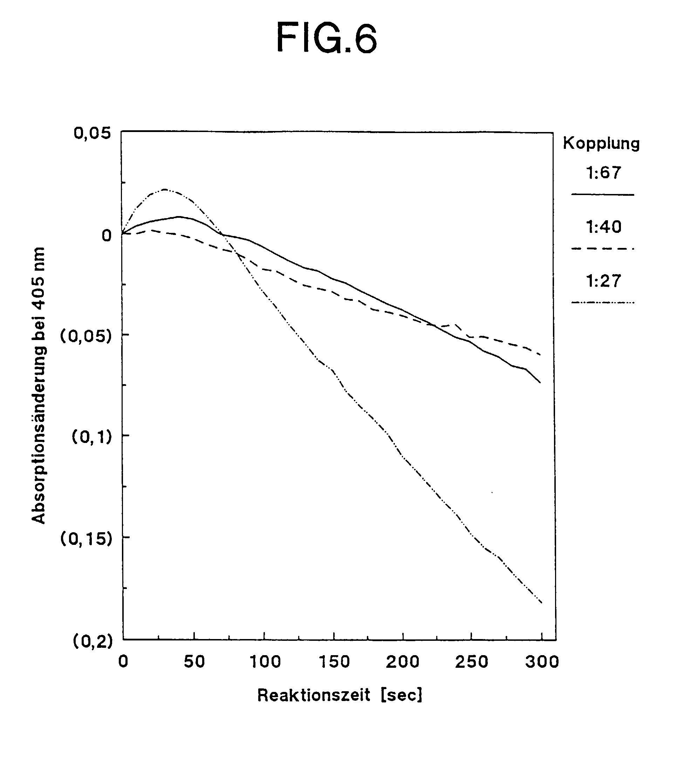

Überraschenderweise zeigte sich auch, daß die Sedimentationsgeschwindigkeit durch die Erhöhung der Beschichtungsdichte ebenfalls verstärkt werden kann. Die beispielsweise in Beispiel 5 ausgeführte Bestimmung von D-Dimer mit Partikeln verschiedener Beladungsdichte, zeigt, wie in Figur 6 dargestellt, daß bei hohen Beladungsdichten die Sedimentationsrate zunimmt. Die erhöhte Reaktivität des Latexreagenzes bedingt jedoch auch eine Trübungszunahme zu Beginn der Reaktion, die durch eine entsprechende Einstellung der Partikelkonzentration, wie im Beispiel 3 ausgeführt, kompensiert werden muß.

Überraschenderweise zeigte sich weiterhin, daß die erfindungsgemäße Bestimmung von Analytkonzentrationen unabhängig vom Aufbau der Meßoptik des Zentrifugalanalysators ist. Als Extremfälle sind die in Beispiel 6 verwendeten Zentrifugalanalysatoren aufgeführt. Während bei einem (z.B. Cobas Bio®) der Lichtweg antiparallel zur Sedimentationsrichtung angeordnet ist, wird im anderen Extremfall (z.B. ACL 300) die Trübung senkrecht zur Sedimentationsrichtung gemessen. In beiden Verfahren ist eine Bestimmung der Analytkonzentrationen nach dem erfindungsgemäßen Verfahren möglich. Somit ist davon auszugehen, daß das erfindungsgemäße Verfahren auf allen Zentrifugalanalysatoren anwendbar ist.

In Beispiel 6 wurde die Bestimmung von D-Dimer vergleichsweise in Medien unterschiedlicher Dichte durchgeführt (Pufferlösung, Serum, Plasma). Der Dichteeffekt führt nur zu geringen Abweichungen der Ergebnisse (Tabellen 1 und 2), so daß dieses Verfahren auch in verschiedenen biologischen Flüssigkeiten problemlos angewandt werden kann.

Das Verfahren ist für alle Analysatoren geeignet, die für das Gemisch aus Probe und Reagenz vor, nach und/oder während der Zentrifugation die optische Dichte ermitteln.

Bevorzugt ist eine Zentrifugalbeschleunigung von 800 - 1.200 x g, wobei die optimale Zentrifugalbeschleunigung eine gewisse Abhängigkeit von der Partikeldichte zeigt.

Als Maß für die Aggregatbildung kann z.B. die Zeit bestimmt werden, bis die im Testansatz bestehende Absorption eine vorgegebene Veränderung erfahren hat (Schwellwert Methode). Eine weitere Methode besteht darin, die Abnahme der Absorption kontinuierlich zu bestimmen und die Rate der Absorptionsabnahme auszuwerten (Kinetische Methode). Schließlich kann auch nach einer bestimmten Zeitdauer die Differenz der Absorption zum Ausgangswert zur Auswertung herangezogen werden (Endpunkt Methode).

Als wasserunlösliche Träger (Festphase) sind die dem Fachmann hinlänglich bekannten natürlichen und synthetischen, organischen und anorganischen Polymere geeignet, wie beispielsweise:

Polystyrol, Polydextrane, Polypropylen, Polyvinylchlorid, Polyvinylidenfluorid, Polyacrylamid, Agarose, Latex, Magnetit, poröses Glaspulver, Erythrozyten, Leukozyten; oder Copolymere aus Styrol-Butadien, Styrol-Methacrylsäure oder Methacrylat-Methacrylsäure, wobei auch Mischpolymere geeignet sind.

Die Träger sind geeigneterweise in partikulärer Form ausgebildet. Als Festphase für den affinen Bindungspartner sind besonders geeignet Polystyrolkugeln oder Latexkugeln.

Bevorzugt sind Partikel mit Durchmessern zwischen 0,1 und 1 µm, besonders bevorzugt mit Durchmessern zwischen 0,1 und 0,3 µm.

Der affine Bindungspartner auf dem Trägermaterial ist bevorzugterweise eine biologische Substanz, beispielsweise ein Antigen, ein Antikörper, ein Hapten, ein Lectin, ein Substratanalogon, ein Proteaseinhibitor oder eine Protease oder inaktive Mutanten hiervon. Bevorzugt sind dabei Antikörper oder Antigene.

Die Konzentration der Partikel wird so gewählt, daß zu Beginn der Messung eine optische Dichte von 5 nicht überschritten wird. Bevorzugt ist eine optische Dichte zwischen 1 und 3.

Zur Detektion wird bevorzugterweise eine Wellenlänge zwischen 280 und 900 nm gewählt. Besonders bevorzugterweise wird eine Wellenlänge verwendet werden, wie sie bei allgemein üblichen chromogenen Assays verwendet und auf klinisch-chemischen Analysatoren zur Verfügung steht, z. B. bei 390, 405, 490, 540, 630 nm. Ganz besonders bevorzugt ist eine Wellenlänge von 405 nm. Bevorzugt sind auch bichromatische Meßverfahren.

Der Testansatz besteht aus der Probe und dem Reagenz, sowie einem Reaktionsmedium. Das Reaktionsmedium kann dem Fachmann bekannte anorganische und organische Substanzen enthalten, die dazu dienen, ein für die Reaktion förderliches Milieu zu schaffen, wie beispielsweise pH-puffernde Substanzen, z.B. Phosphat, Tris(hydroxymethyl)aminomethan, Dichte und Viskosität beeinflussende Substanzen, z. B. Polyethylenglycol, Glycerin, oder Substanzen, die Störungen durch Lipide, z.B. Detergenzien, oder Rheumafaktoren, z. B. gamma-Globulin Fraktionen, minimieren. Bevorzugterweise besteht das Reaktionsmedium aus einer physiologischen NaCl-Lösung.

Die Bestimmung erfolgt vorzugsweise bei +10° bis 40°C, besonders bevorzugterweise bei +20° bis +40°C, ganz besonders bevorzugterweise bei +37°C.

Das erfindungsgemäße Verfahren zeichnet sich aus durch seine Einfachheit, die leichte Automatisierbarkeit und eine gegenüber den bisher bekannten, turbidimetrischen Verfahren verbesserte Meßpräzision.

Die Erfindung soll durch die folgenden Beispiele näher beschrieben, in keiner Weise jedoch eingeschränkt werden.

Die in den Beispielen angegebenen Prozentwerte für die Partikelkonzentrationen sind jeweils Gewichtsprozente.

### Beispiel 1:

### Herstellung eines Latexreagenz zur Bestimmung von Fibrinfragment D-Dimer

Die Herstellung von Latexreagenzien erfolgte nach W. H. Kapmeyer et al., "Automated nephelometric immunoassays with novel shell/core particles", J.Clin.Lab.Anal. 2, 76 - 83 (1988). Die Kopplung eines monoklonalen Antikörpers gegen D-Dimer (MAk DD5, Behringwerke AG, Marburg, FRG) an die Dipentylacetal-Gruppen des Pfropfpolymerisats erfolgte im Verhältnis von 1:67 bis 1:29, bezogen auf das Gewicht der Reaktionspartner.

1 ml des Pfropfpolymerisats (4 %) wurde mit 0,1 ml einer MAk DD5 Lösung (1 mg/ml; entspricht einem Kopplungsverhältnis von 1:40) und 0,05 ml einer 20 %igen wässrigen Tween^{R} 20 Lösung gemischt. Zur Aktivierung des Latex wurde die Lösung mit ca. 0,01 ml einer 1 N HCl-Lösung auf einen pH von 2 eingestellt. Nach einer 30-minütigen Inkubation bei Raumtemperatur wurden 0,25 ml einer gesättigten Natriumhydrogenphosphat-Lösung (pH 6,5) und 0,25 ml einer wässrigen Natriumborhydrid-Lösung (25 mg/ml) hinzugefügt und intensiv gemischt. Die Kopplung des Antikörpers an die aktivierten Aldehydgruppen erfolgte über 1 h bei Raumtemperatur. Anschließend wurde das anti-D-Dimer Latex Konjugat zentrifugiert (Beckman Zentrifuge, 40000 x g, 30 Minuten) und das Pellet in 1,5 ml eines 0,1 molaren Glycin-Puffers (pH 8,2; enthaltend 0,17 M NaCl und 0,5 % Tween^{R} 20) resuspendiert. Die Lösung wurde mit Ultraschall für ca. 5 sec behandelt (Branson Sonifier B 15). Diese Stammlösung wurde bei +4 °C gelagert. Arbeitslösungen in Konzentrationen von 0,025 bis 0,2 %, bezogen auf den Latexfeststoffgehalt, wurden durch Verdünnung der Stammlösung mit physiologischer Kochsalzlösung hergestellt (entspricht 0,0125 - 0,1 % in der Reaktionslösung).

### Beispiel 2:

### Kinetik der Trübungssignale bei Verwendung von Latexreagenzien in bisher üblichen Verfahren

Ein handelsübliches Latexreagenz zur nephelometrischen Bestimmung von IgE in Serum und ein IgE-Standard Serum mit einer Konzentration von 512 IU/ml (NA-Latex-IgE Reagenz, Behringwerke AG, Marburg, FRG) wurden vorschriftsmäßig mit destilliertem Wasser rekonditioniert. Der IgE Standard wurde unverdünnt und in Verdünnungen mit physiologischer Kochsalzlösung eingesetzt. Ein nach Beispiel 1 hergestelltes D-Dimer Latexreagenz mit einem Kopplungsverhältnis von 1:40 wurde mit physiologischer Kochsalzlösung auf eine Konzentration von 0,025 % verdünnt. Ein D-Dimer Standard (Behringwerke AG, Marburg, FRG) wurde mit physiologischer Kochsalzlösung auf Konzentrationen von 62,5 bis 500 µg/l verdünnt. Als Reaktionsmedium wurde physiologische Kochsalzlösung verwendet.

Die Messung erfolgte in einem Zentrifugalanalysator (Modell Cobas Bio®, F. Hoffmann La Roche & Co.AG, Basel, Schweiz). 40 µl Probe und 5 µl Reaktionsmedium wurden vorgelegt. Nach 10 sec wurden 40 µl Latexreagenz hinzugefügt. Die Reaktion wurde durch das Mischen der Proben mittels Zentrifugation gestartet. Bei konstanter Zentrifugationsgeschwindigkeit von ca. 1000 x g wurde die Absorption bei 405 nm in Intervallen von 10 sec über eine Zeitdauer von 5 min gemessen.

Die Absorption zu Beginn der Messung betrug ca. 0,8 Einheiten. Die Änderung des anfänglichen Absorptionssignals über den Meßzeitraum bei 405 nm ist in Figur 1 für das IgE-Latexreagenz, in Figur 2 für das D-Dimer-Latexreagenz dargestellt.

### Beispiel 3:

### Abhängigkeit der Reaktionskinetik von der Partikeldichte im Meßansatz.

Ein nach Beispiel 1 hergestelltes anti-D-Dimer Latexreagenz mit einem Kopplungsverhältnis von 1:40 wurde mit physiologischer Kochsalzlösung auf Konzentrationen von 0,025, 0,1 und 0,2 % verdünnt. Ein D-Dimer Standard (Behringwerke AG, Marburg, FRG) wurde mit physiologischer Kochsalzlösung auf eine Konzentration von 250 µg/l verdünnt. Als Reaktionsmedium wurde physiologische Kochsalzlösung verwendet.

Die Bestimmung der Absorptionsänderung erfolgte am Zentrifugalanalysator wie unter Beispiel 2 beschrieben. Die Extinktionen zu Beginn der Messung betrugen 0,8, 1,4 und 1,8 Einheiten bei 405 nm. Der Verlauf der bestimmten Absorptionsänderung ist in Figur 3 dargestellt.

### Beispiel 4:

### Verlauf der Trübungssignale bei Verwendung von Latexreagenzien im erfindungsgemäßen Verfahren

Ein lyophilsiertes IgE-Latexreagenz (NA-Latex-IgE Reagenz, Behringwerke AG, Marburg, FRG) wurde mit nur 1/3 der normalerweise verwendeten Menge an destilliertem Wasser rekonditioniert. Ein nach Beispiel 1 hergestelltes anti-D-Dimer Latexreagenz mit einem Kopplungsverhältnis von 1:40 wurde mit physiologischer Kochsalzlösung auf eine Konzentration von 0,2 % verdünnt. IgE- und D-Dimer Standards wurden wie in Beispiel 2 beschrieben verwendet.

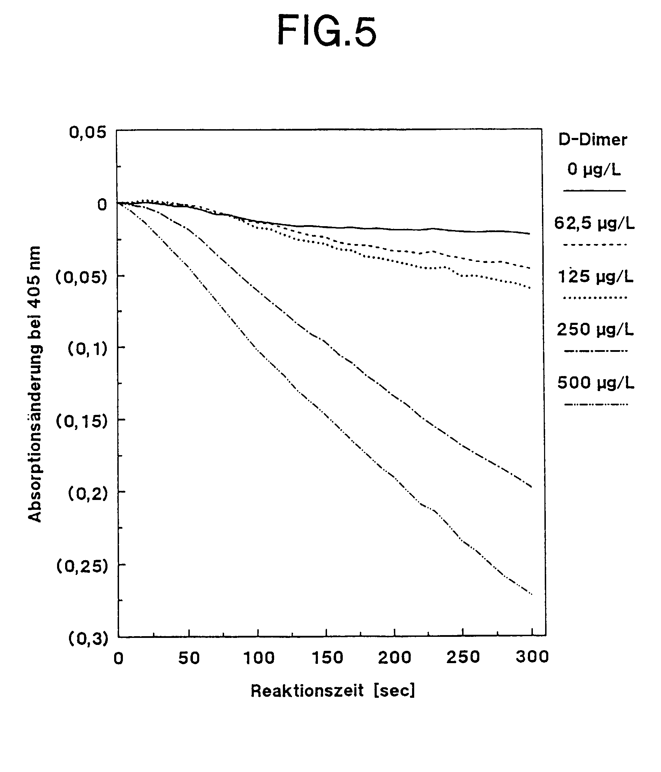

Die Bestimmung der Absorptionsänderung erfolgte am Zentrifugalanalysator wie unter Beispiel 2 beschrieben. Die Extinktionen zu Beginn der Messung betrugen ca. 1,8 Einheiten bei 405 nm. Der Verlauf der bestimmten Absorptionsänderung bei verschiedenen Antigenkonzentrationen ist in Figur 4 für das IgE-, in Figur 5 für das D-Dimer-Latexreagenz dargestellt.

### Beispiel 5:

### Abhängigkeit der Reaktionskinetik von der Beladungsdichte des Latexreagenzes

Nach Beispiel 1 hergestellte anti-D-Dimer Latexreagenzien mit Kopplungsverhältnissen von 1:67, 1:40 und 1:29 wurden mit physiologischer Kochsalzlösung auf eine Konzentration von 0,2 % verdünnt. Ein D-Dimer Standard (Behringwerke AG, Marburg, FRG) wurde mit physiologischer Kochsalzlösung auf eine Konzentration von 125 µg/l verdünnt. Als Reaktionsmedium wurde physiologische Kochsalzlösung verwendet.

Die Bestimmung der Absorptionsänderung erfolgte am Zentrifugalanalysator wie unter Beispiel 2 beschrieben. Die Absorption der Lösung zu Beginn der Messung betrug ca. 1,8 Einheiten bei 405 nm. Der Verlauf der bestimmten Absorptionsänderungen bei Latexreagenzien unterschiedlicher Antikörperbeladung ist in Figur 6 dargestellt.

### Beispiel 6:

### Bestimmung von D-Dimer in Phosphatpuffer, Serum und Plasma in einem Zentrifugalanalysator mit senkrecht zur Sedimentationsrichtung angeordneten Meßstrahl

Das gemäß Beispiel 1 beschichtete Latexreagenz (Kopplungsverhältnis 1:29) wurde in phosphatgepufferter, isotonischer Natriumchloridlösung (PBS) auf eine Arbeitskonzentration von 0,2% verdünnt. D-Dimer Antigen (Behringwerke AG, Marburg, FRG) wurde PBS oder einem Pool aus Serum bzw. Plasma von normalen Blutspendern zugesetzt, so daß die Endkonzentrationen 0, 313, 625, 1250, 2500 und 5000 µg/l betrugen. Als Reaktionsmedium wurde PBS verwendet.

Die Messung erfolgte in einem Zentrifugalanalysator des Typs ACL 300 der Firma Instrument Laboratory, Mailand, Italien. In diesem Analysator verläuft der Meßstrahl senkrecht zur Sedimentationsrichtung durch die Probenlösung. 20 µl Probe (PBS, Serum oder Plasma) und 80 µl Reaktionsmedium wurden vorgelegt und durch Zentrifugation gemischt. Nach 30 sec wurden 50 µl Latexreagenz hinzupipettiert. Die Reaktion erfolgte anschließend bei ca. 800 x g. Die Messung erfolgte kontinuierlich bei 405 nm über 10 min.

Das Gerät bestimmt den Abfall der Lichtabsorption gegenüber einer gerätespezifischen Referenz lösung und stellt die Ergebnisse in positiver Notation dar. Die Auswertung nach verschiedenen Methoden (Endpunkt, Kinetische und Schwellwert Methode) ist in Tabelle 1 aufgeführt. Die gefundene Zeit, bis ein Absorptionsabfall um 0,1 eingetreten ist, ist umgekehrt proportional zur D-Dimer Konzentration. Die Rate des Absorptionsabfalls, bzw. die Gesamtabsorptionsabnahme am Ende der Messung ist direkt proportional zur D-Dimer Konzentration in der Probe. Die Methode führt sowohl in PBS, als auch in Serum und Plasma zu übereinstimmenden Ergebnissen.

### Tabelle 1

Tabelle 1 zeigt die Abhängigkeit der Absorptionsänderungen in einem Zentrifugalanalysator mit senkrecht zur Sedimentationsrichtung verlaufenden Lichtgang.

**Tabelle 1**

| Endpunkt Methode: Absorption(t=0 sec.)-Absorption (t=300 sec.) | | | |
|---|---|---|---|
| D-Dimer [µg/l] | PBS | Serum | Plasma |
| 0 | -0,013 | -0,004 | 0,003 |
| 313 | 0,194 | 0,185 | 0,213 |
| 625 | 0,423 | 0,406 | 0,447 |
| 1250 | 0,702 | 0,629 | 0,627 |
| 2500 | 0,874 | 0,775 | 0,748 |
| 5000 | 0,926 | 0,838 | 0,796 |

| Kinetische Methode: Absorptionsänderung pro min in [- 1/min] | | | |
|---|---|---|---|
| D-Dimer [µg/l] | PBS | Serum | Plasma |
| 0 | 0,005 | 0,005 | 0,005 |
| 313 | 0,068 | 0,069 | 0,119 |
| 625 | 0,207 | 0,192 | 0,205 |
| 1250 | 0,296 | 0,245 | 0,254 |
| 2500 | 0,324 | 0,273 | 0,281 |
| 5000 | 0,333 | 0,295 | 0,298 |

| Schwellwert Methode: Zeit bis Absorptionsänderung <-0,1 [sec] | | | |
|---|---|---|---|
| D-Dimer [µg/l] | PBS | Serum | Plasma |
| 0 | >600 | >600 | >600 |
| 313 | 389 | 416 | 431 |
| 625 | 327 | 347 | 349 |
| 1250 | 289 | 301 | 306 |
| 2500 | 264 | 279 | 289 |
| 5000 | 259 | 271 | 278 |

### Beispiel 7

### Bestimmung von D-Dimer in Phosphatpuffer und Plasma in einem Zentrifugalanalysator mit antiparallel zur Sedimentationsrichtung angeordneten Meßstrahl

Das gemäß Beispiel 1 beschichtete D-Dimer-Latexreagenz (Kopplungsverhältnis 1:29) wurde in phosphatgepufferter, isotonischer Natriumchloridlösung (PBS) auf eine Arbeitskonzentration von 0,2 % eingestellt. Dies entspricht einer Absorption von ca. 1,8 bei einer Wellenlänge von 405 nm. D-Dimer Antigen (Behringwerke AG, Marburg, FRG) wurde PBS oder einem Pool aus Plasma von normalen Blutspendern zugesetzt, so daß die Endkonzentrationen 0, 62.5, 125, 250, 500, 1000 bzw. 2000 µg/l betrugen. Als Reaktionsmedium wurde PBS verwendet.

Die Messung erfolgte in einem Zentrifugalanalysator des Typs Cobas Bio® (F. Hoffmann La Roche & Co.AG, Basel, Schweiz), wie in Beispiel 2 beschrieben. In diesem Analysator verläuft der Meßstrahl antiparallel zur Sedimentationsrichtung.

Die Auswertung nach der Endpunkt, Kinetischen und der Schwellwert Methode ist in Tabelle 2 aufgeführt. Die gefundene Zeit bis ein Absorptionsabfall um 0,05 eingetreten ist, ist umgekehrt proportional zur D-Dimer Konzentration. Die Rate des Absorptionsabfalls, bzw. die Gesamtabsorptionsabnahme am Ende der Messung ist direkt proportional zur D-Dimer Konzentration in der Probe. Die Methode führt in PBS und Plasma zu übereinstimmenden Ergebnissen.

### Tabelle 2

Tabelle 2 zeigt die Abhängigkeit der Absorptionsänderungen in einem Zentrifugalanalysator mit anti-parallel zur Sedimentationsrichtung verlaufendem Lichtgang.

**Tabelle 2**

| Endpunkt Methode: Absorption(t=0 sec.)-Absorption (t=300 sec.) | | |
|---|---|---|
| D-Dimer [µg/l] | PBS | Plasma |
| 0 | 0,022 | 0,020 |
| 62,5 | 0,045 | 0,075 |
| 125 | 0,059 | 0,131 |
| 250 | 0,197 | 0,205 |
| 500 | 0,272 | 0,250 |
| 1000 | 0,341 | 0,321 |
| 2000 | 0,343 | 0,347 |

| Kinetische Methode: Absorptionsänderung pro min in [- 1/min] | | |
|---|---|---|
| D-Dimer [µg/l] | PBS | Plasma |
| 0 | 0,0028 | 0,0040 |
| 62,5 | 0,0087 | 0,0172 |
| 125 | 0,0148 | 0,0284 |
| 250 | 0,0359 | 0,0422 |
| 500 | 0,0485 | 0,0552 |
| 1000 | 0,0773 | 0,0884 |
| 2000 | 0,0755 | 0,0967 |

| Schwellwert Methode: Zeit bis Absorptionsänderung <-0,05 [sec] | | |
|---|---|---|
| D-Dimer [µg/l] | PBS | Plasma |
| 0 | >300 | >300 |
| 62,5 | 300 | 200 |
| 125 | 240 | 120 |
| 250 | 80 | 70 |
| 500 | 50 | 50 |
| 1000 | 40 | 40 |
| 2000 | 30 | 40 |

**Legenden zu den Figuren:**

### Figur 1:

Kinetik der Reaktion anti-IgE beschichteter Latexpartikel eines handelsüblichen Präparates mit verschiedenen Konzentrationen von IgE in Serum bei üblicherweise verwendeten Latexkonzentrationen. Die Trübung wurde bei 405 nm in Cobas Bio über 5 Minuten in Abständen von 10 Sekunden gemessen. Die Konzentration des Reagenzes betrug 0,025% in der Probenlösung. Dargestellt sind die Meßdifferenzen zum Absorptionswert nach 0,5 Sekunden.

### Figur 2:

Kinetik der Reaktion anti-D-Dimer beschichteter Latexpartikel mit verschiedenen Konzentrationen von D-Dimer in physiologischer Kochsalzlösung bei üblicherweise verwendeten Latexkonzentrationen. Die Trübung wurde bei 405 nm in Cobas Bio über 5 Minuten in Abständen von 10 Sekunden gemessen. Die Konzentration des Reagenzes betrug 0,025% in der Probenlösung. Das Kopplungsverhältnis (Antikörper:Latex) des Reagenzes war 1:40. Dargestellt sind die Meßdifferenzen zum Absorptionswert nach 0,5 Sekunden.

### Figur 3:

Abhängigkeit des Trübungssignals von der Dichte sensibilisierter Partikel im Reaktionsmedium. Mit anti-D-Dimer beschichtete Latexpartikel (Kopplungsverhältnis 1:40) wurden in physiologischer Kochsalzlösung auf Konzentrationen von 0,025, 0,1 und 0,2% verdünnt. Die Reaktionskinetik mit einer D-Dimer Lösung (250 µg/l)wurde bei 405 nm im Cobas Bio über 5 Minuten in Abständen von 10 Sekunden gemessen. Dargestellt sind die Meßdifferenzen zum Absorptionswert nach 0,5 Sekunden.

### Figur 4:

Kinetik der Reaktion anti-IgE beschichteter Latexpartikel eines handelsüblichen Präparates mit verschiedenen Konzentrationen von IgE in Serum nach dem erfindungsgemäßen Verfahren. Die Trübung wurde bei 405 nm in Cobas Bio über 5 Minuten in Abständen von 10 Sekunden gemessen. Die Konzentration des Reagenzes betrug 0,2% in der Probenlösung. Dargestellt sind die Meßdifferenzen zum Absorptionswert nach 0,5 Sekunden.

### Figur 5:

Kinetik der Reaktion anti-D-Dimer beschichteter Latexpartikel mit verschiedenen Konzentrationen an D-Dimer in physiologischer Kochsalzlösung nach dem erfindungsgemäßen Verfahren. Die Trübung wurde bei 405 nm in Cobas Bio über 5 Minuten in Abständen von 10 Sekunden gemessen. Die Konzentration des eingesetzten Reagenzes (Kopplungsverhältnis 1:40) betrug 0,2%. Dargestellt sind die Meßdifferenzen zum Absorptionswert nach 0,5 Sekunden.

### Figur 6:

Abhängigkeit des Trübungssignals von der Beladungsdichte von Latexpartikeln mit anti-D-Dimer Antikörpern. Latexpartikel wurden nach dem im Beispiel 1 beschriebenen Verfahren mit einem monoklonalen Antikörper gegen D-Dimer im Verhältnis von 1:67, 1:40 und 1:29 beschichtet. Die Reagenzien wurden in einer Konzentration von 0,2% eingesetzt. Dargestellt sind die Meßdifferenzen zur Absorption nach 0,5 Sekunden, die mit einer D-Dimer Lösung (125 µg/l) bei 405 nm im Cobas Bio über 5 Minuten in Abständen von 10 Sekunden gemessen wurden.

## Patentansprüche

1. Verfahren zur Bestimmung von Analyten, wobei eine Probe eines biologischen Materials, die möglicherweise diesen Analyten enthält mit mindestens einem für den Analyten spezifischen Bindungspartner, der an einem partikelförmigen Trägermaterial immobilisiert ist, inkubiert wird, dadurch gekennzeichnet, daß
a) die Reaktion unter Einfluß einer im Meßverlauf konstanten Zentrifugalbeschleunigung erfolgt, die zwischen 800 und 10.000 x g liegt,
b) die Partikeldichte im Meßansatz mindestens 0,1 Gew. % beträgt,
c) als Trägermaterial möglichst kugelförmige, partikuläre, natürliche und/oder synthetischen Polymere verwendet werden,
d) die Reaktion in einem gepufferten Medium mit definierter Dichte und Viskosität erfolgt,
e) die auftretende Trübungsaufhellung bereits maximal 1 min nach der Probenzugabe gemessen wird.

2. Verfahren nach Anspruch 1, dadurch gekennzeichnet, daß Analyt und spezifischer Bindungspartner Partner einer immunchemischen Reaktion sind.

3. Verfahren nach Anspruch 1, dadurch gekennzeichnet, daß die Veränderung der Absorption in einem Winkel von 0° bis 180° zur Sedimentationsrichtung erfolgen kann.

4. Verfahren nach Anspruch 1, dadurch gekennzeichnet, daß zur Messung der Absorptionsänderung Wellenlängen aus dem Bereich von 280 bis 900 nm verwendet werden.

5. Verfahren nach Anspruch 1, dadurch gekennzeichnet, daß die Zentrifugalbeschleunigung in dem Bereich zwischen 800 - 2.000 x g, bevorzugterweise 800 - 1,200 x g, liegt.

6. Verfahren nach Anspruch 1, dadurch gekennzeichnet, daß als spezifischer Bindungspartner ein Antikörper gegen das Fibrinspaltprodukt D-Dimer verwendet wird.

7. Verfahren nach Anspruch 1, dadurch gekennzeichnet, daß als partikelförmiges Trägermaterial Copolymerisate aus Styrol-Methacrylsäure oder Methacrylat-Methacrylsäure verwendet werden.

8. Verfahren nach Anspruch 1, dadurch gekennzeichnet, daß die Trübungsaufhellung bereits maximal 10 sec nach der Probenzugabe gemessen wird.

## Claims

1. A method for determining analytes, in which a sample of a biological material which possibly contains this analyte is incubated with at least one binding partner which is specific for the analyte and which is immobilized on a particulate carrier material, wherein
a) the reaction takes place under the influence of a centrifugal acceleration which is constant during the course of the measurement and which is between 800 and 10,000 x g,
b) the particle density in the mixture for measurement is at least 0.1 % by weight,
c) particulate natural and/or synthetic polymers which are as spherical as possible are used as carrier material,
d) the reaction takes place in a buffered medium with defined density and viscosity,
e) the lightening in turbidity which occurs is measured at most 1 min after the addition of the sample.

2. The method as claimed in claim 1, wherein analyte and specific binding partner are partners in an immunochemical reaction.

3. The method as claimed in claim 1, wherein the change in the absorption can take place at an angle of 0° to 180° to the direction of sedimentation.

4. The method as claimed in claim 1, wherein wavelengths from the range from 280 to 900 nm are used to measure the change in absorption.

5. The method as claimed in claim 1, wherein the centrifugal acceleration is in the range between 800 and 2000 x g, preferably 800 - 1200 x g.

6. The method as claimed in claim 1, wherein an antibody against the fibrin degradation product D dimer is used as specific binding partner.

7. The method as claimed in claim 1, wherein styrene/ methacrylic acid or methacrylate/methacrylic acid copolymers are used as particulate carrier material.

8. The method as claimed in claim 1, wherein the lightening in turbidity is measured at most 10 sec after the addition of the sample.

## Revendications

1. Procédé de dosage d'analytes, dans lequel on incube un échantillon d'un matériau biologique susceptible de contenir ces analytes avec au moins un partenaire de liaison spécifique de l'analyte qui est immobilisé sur un matériau support particulaire, caractérisé en ce que
a) la réaction se fait sous l'influence d'une accélération centrifuge constante au cours de la mesure et comprise entre 800 et 10 000 x g,
b) la densité des particules dans la charge de mesure est d'au moins 0,1 % en poids,
c) on utilise comme matériau support des polymères naturels ou synthétiques particulaires, éventuellement en forme de billes,
d) la réaction se fait dans un milieu tamponné de densité et de viscosité définies,
e) l'élimination de la turbidité formée est mesurée au maximum 1 minute après l'ajout de l'échantillon.

2. Procédé selon la revendication 1, caractérisé en ce que l'analyte et le partenaire de liaison spécifique sont des partenaires d'une réaction immunochimique.

3. Procédé selon la revendication 1, caractérisé en ce que la variation de l'absorption peut se produire dans un angle de 0° à 180° par rapport à la direction de la sédimentation.

4. Procédé selon la revendication 1, caractérisé en ce que l'on utilise pour la mesure de la variation de l'absorption des longueurs d'ondes dans la plage de 280 à 900 nm.

5. Procédé selon la revendication 1, caractérisé en ce que l'accélération centrifuge se situe dans la plage de 800 à 2 000 x g, de préférence de 800 à 1 200 x g.

6. Procédé selon la revendication 1, caractérisé en ce que l'on utilise comme partenaire de liaison spécifique un anticorps dirigé contre le dimère D produit de dissociation de la fibrine.

7. Procédé selon la revendication 1, caractérisé en ce que l'on utilise comme matériau support particulaire des copolymères de styrène et d'acide méthacrylique ou de méthacrylate et d'acide méthacrylique.

8. Procédé selon la revendication 1, caractérisé en ce que l'élimination de la turbidité formée est mesurée au maximum 10 secondes après l'ajout de l'échantillon.
